# EUROPEAN PATENT SPECIFICATION

(11) **EP 1 182 963 B1**
(45) Date of publication and mention of the grant of the patent: **16.03.2005**
(21) Application number: 00936039.7
(22) Date of filing: 18.05.2000
(51) Int. Cl.: A61B 5/00, A61B 1/00

(54) **OPTICAL BIOPSY SYSTEM**
OPTISCHES BIOPSIESYSTEM
SYSTEME DE BIOPSIE OPTIQUE

(30) Priority: 18.05.1999 US 134716 P
(43) Date of publication of application: 06.03.2002
(73) Proprietor: Boston Scientific Limited, St. Michael, Barbados, West Indies (BB)
(72) Inventor: LENNOX, Charles, D., Hudson, NH 03051 (US)
(74) Representative: Lock, Graham James
(86) International application number: PCT/US2000/013642
(87) International publication number: WO 2000/069332

(56) References cited:
- EP-A- 0 520 743
- WO-A-99/18844
- US-A- 5 337 734
- US-A- 5 496 259
- US-A- 5 630 782
- US-A- 5 852 494
- US-A- 5 855 551

## Description

### RELATED APPLICATIONS

This application claims benefit of priority to U.S. provisional patent application Ser. No. 60/134,716, filed May 18, 1999.

### FIELD OF THE INVENTION

The invention relates generally to probe sheaths and probe tips for use with interventional devices which include tissue spectroscopy or optical biopsy devices. More particularly, the invention relates to probe tips which are adapted to particular tissue spectroscopy or optical biopsy applications within the body, and which are wholly or partly disposable, and which are wholly or partly interchangeable.

### BACKGROUND OF THE INVENTION

Tissue spectroscopy or optical biopsy procedures constitute a relatively new field in which radiation waves, including visible light, ultraviolet light, infrared radiation, or X-rays, are used to detect disease, infection or other conditions in the human body. Systems for performing such procedures typically include one or more light sources that illuminate the tissue, and one or more receiving channels that measure or map the resulting emission from the illuminated tissue. Interventional devices for performing such procedures can take on many forms. A common form is a long fiber-equipped catheter with a proximally mounted connector and a distal tip that is capable of sending and receiving light through optical transmission. Another form is a probe, which can contain a light source and detector, that can be placed in a body cavity. Examples of such probes are provided in commonly owned, copending U.S. Pat. Appln. Ser. No. 08/898,604, entitled "Miniature Spectrometer"

Within these probe-style spectroscopic devices, it is possible to include an entire power supply, light emission system, light detection system, and readout system. Such probes can be made disposable using mass production techniques, but certain elements are expensive and preferably reusable.

In situations in which repeated use of the probe is desirable, such as when repeated calibrations are required, or when repeated application of the probe to various areas of the body is desired, disposable outer sheaths may be employed, which reduce problems of probe contamination and disease transfer. It has been suggested that condoms can be used as a sheath over such probes to prevent the occurrence of these problems. However, condoms and many other known sheath materials often attenuate the radiation transmitted in the visible and ultraviolet wavelength ranges that are most useful diagnostically. Therefore, sheaths have been specifically designed for use with interventional devices that are substantially transmissive of the wavelengths of radiation used in tissue spectroscopy or optical biopsy. Examples of such sheaths are provided in commonly owned, copending U.S. Pat. Appln. Ser. No. 08/939,707, entitled "Sheath for Tissue Spectroscopy"

US 5630782 discloses a combination probe sheath and probe tip product for use with an interventional device, comprising a probe sheath having an open proximal end and a distal end; and a probe tip disposed at the distal end wherein at least a portion of the probe tip is substantially transmissive to radiation.

US 5852494 discloses a calibration device for use with an interventional device employing a source of radiation, comprising a body defining a cavity adapted to receive a probe tip of said device and a coating on a surface of the cavity that absorbs or reflects radiation at predetermined levels to calibrate said interventional device.

### SUMMARY OF THE INVENTION

A system for use with interventional devices is disclosed as defined in claim 1 which include improved probe sheaths and probe tips for tissue spectroscopy or optical biopsy. In particular, probe sheaths and probe tips are disclosed which are adapted to particular tissue spectroscopy or optical biopsy applications within the body, and which are wholly or partly disposable, and which are wholly or partly interchangeable.

Thus, in one aspect, the present invention provides probe sheaths and probe tips for use with an interventional device, which are substantially transmissive of wavelengths used in tissue spectroscopy or optical biopsy, and which are adapted for particular applications within the body. The invention also provides a system of disposable and/or interchangeable probe sheaths and probe tips. Thus, either a one-piece probe sheath and probe tip assembly is provided which is disposable, or a two-piece probe sheath and probe tip assembly is provided in which at least the probe tip is disposable. Alternatively, or in addition, a two-piece probe sheath and probe tip assembly is provided in which the probe sheath is adapted for use with a certain type of probe (e.g., cervical, hysterscopic, bladder, ureteral, colonoscopic, sigmoidoscopic, esophageal, or other endoscopic) and the probe tip is adapted for a particular application within the body (e.g., scatter tip, deflectional tip, smear tip, brush tip, insertional tip, or dye tip). Thus, the probe sheath may be constructed with a distal probe tip that is integral with the probe sheath, and the integral probe sheath and tip may have application specific characteristics and be optionally disposable. Alternatively, the probe sheath may be constructed such that a distal probe tip is removable and replaceable. This feature allows for interchange of probe tips to prevent contamination from one tissue interrogation site to another, or for interchange of probe tips which have differing functional characteristics, and one or both of the probe sheath and probe tip may be disposable.

Methods of using the probe sheaths and probe tips of the invention, kits comprising the probe sheaths and probe tips of the invention, and interventional device systems comprising the probe sheaths and probe tips of the invention, are also provided.

### BRIEF DESCRIPTION OF THE DRAWINGS

FIGURE 1 is a representation of one embodiment of an interventional device and system of the invention in an exploded view.
FIGURE 2 is a representation of one embodiment of an interventional device of the invention.
FIGURE 3 is a representation of one embodiment of a probe of the invention, including a substantially straight probe assembly and probe sheath.
FIGURE 4 is a representation of one embodiment of a probe of the invention, including a substantially straight probe assembly and probe sheath with a deflectable distal portion which is just proximal to the probe tip.
FIGURE 5 is a representation of one embodiment of a probe of the invention, including a flexible probe assembly and probe sheath, and entry ports distal to the handle assembly but proximal to the probe sheath for introduction of additional interventional device elements.
FIGURE 6 is a representation of one embodiment of a probe of the invention, including a substantially straight probe assembly and probe sheath with a deflectable distal portion which is just proximal to the probe tip, and entry ports distal to the handle assembly but proximal to the probe sheath for introduction of additional interventional device elements.
FIGURE 7 is a representation of one non-claimed embodiment of a probe tip for an interventional device, including a substantially convex exterior surface for radiation scattering and collection.
FIGURE 8 is a representation of one non-claimed embodiment of a probe tip for an interventional device, including a radially oriented window or aperture through which the radiation beam may be deflected.
FIGURE 9 is a representation of one non-claimed embodiment of a probe tip for an interventional device, including an extension of substantially smaller diameter than the main portion of the probe and sheath, designed for accessing smaller body cavities, orifices, perforations, incisions, or other apertures.
FIGURE 10 is a representation of one embodiment of a probe tip for an interventional device of the invention, including raised projections with angular surfaces for dislodging or abrading tissue samples.
FIGURE 11 is a representation of one embodiment of a probe tip for an interventional device of the invention, including substantially cylindrical projections for dislodging or abrading tissue samples and/or transmitting radiation to or from sites in contact with the ends of the projections.
FIGURE 12 is a representation of one non-claimed embodiment of a probe tip for an interventional device engaged with a calibration cap for calibrating the signals received by the interventional device.

### DETAILED DESCRIPTION OF THE INVENTION

The present invention depends, in part, upon the development of new designs for probe sheaths and probe tips for interventional devices used in tissue spectroscopy or optical biopsy. The probe sheaths and probe tips of the invention possess the advantageous characteristics of being adapted to particular applications within the body, being wholly or partly disposable, and being wholly or partly interchangeable. Prior to the present invention, probe sheaths and probe tips were designed for general use and were not specifically adapted for particular applications within the body. The tissue spectroscopy or optical biopsy systems described herein offer more economical and effective means of conducting routine screening, detecting cancers or precursors to cancer, and conducting definitive diagnoses during operative or other interventional procedures.

### Definitions.

In order to more clearly and concisely describe and point out the subject matter of the claimed invention, the following definitions are provided for specific terms which are used in the following written description and the appended claims.

As used herein, the term "interventional device" means any medical device which is placed or inserted into the body through an orifice, incision, or other aperture in order to introduce sampling, testing, diagnostic, surgical or therapeutic devices. Such interventional devices can include a variety of different devices in combination for achieving multiple tasks. Thus, for example, interventional devices may include, without limitation, catheters, cauterizers, endoscopes, guide wires, needles, electrodes, fiber optics, imaging devices, microwave antennas, ultrasound probes, cannulas, stents, clamps, scissors, staplers, forceps, scalpels, snares, angioplasty balloons, vacuums, or introducers.

As used herein, the term "probe" means that portion of an interventional device which normally may be placed or inserted into the body, including the probe assembly, probe sheath and probe tip, but excludes that portion which normally remains outside of the body, such as the handle assembly or control and display assembly.
As used herein, the term "spectroscopy" means the use of an instrument which produces radiation directed at a target and which then measures the radiation which is emitted in response by the target. Modes of spectroscopy used by the system may include, without limitation, visible light spectroscopy, infrared spectroscopy, ultraviolet spectroscopy, autofluorescence, dye-enhanced light-induced fluorescence, Raman scatter spectroscopy, elastic scatter spectroscopy, X-ray spectroscopy.

### Description of Preferred Embodiments

The present invention relates to an interventional device for use in a tissue spectroscopy or optical biopsy system. The tissue spectroscopy or optical biopsy system provides for the detection of tissue condition at the skin surface, at the inner or outer surface of a hollow organ or vessel, at an interstitial space in a solid organ, or within any other perforation, space or other aperture which occurs naturally or which has been produced by surgery or trauma.

The probe assemblies, probe sheaths and probe tips are configured for specific anatomical sites, for the means to access the anatomical sites, for the type of tissue at the anatomical site, and for the conditions of interest for that tissue at the anatomical site. The probes may be rigid or flexible, may be used directly, or may be used through the working channel of an endoscope.
The probes may also be constructed to determine the condition of tissue ex vivo.

### Tissue Spectroscopy or Optical Biopsy System

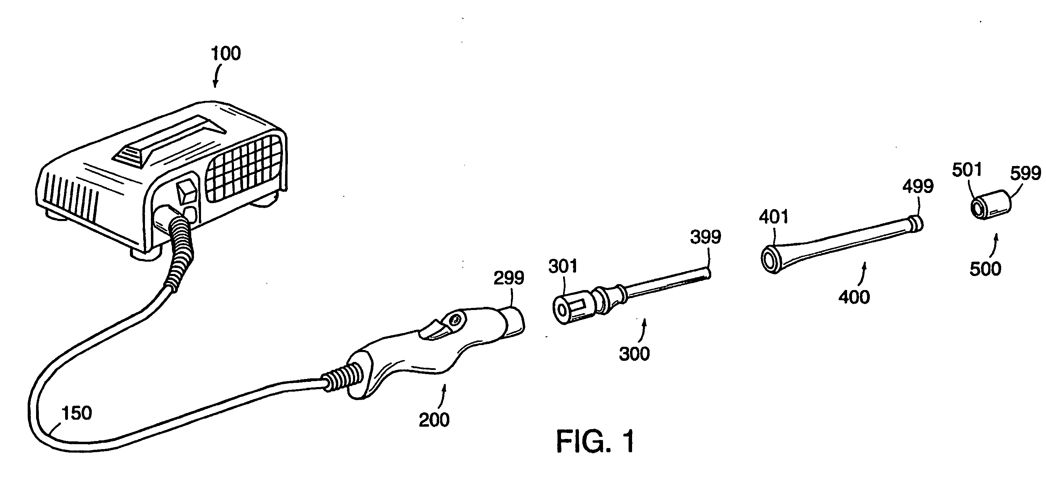

As shown in Figure 1, the system comprises a control and display assembly 100 which may be common to all applications, a handle assembly 200 which may also be common to all applications, a cable 150 connecting the control and display assembly 100 to the handle assembly 200, a reusable probe assembly 300 that may be adapted in size, shape and flexibility for particular applications within the body, a probe sheath 400 that is adapted to fit over at least the distal portion of the probe assembly 300, and a probe tip 500 that may be integral with the probe sheath or which may be connected to the distal end 499 of the probe sheath 400 by the user and which, therefore, may be interchanged for different applications within the body.

Thus, in preferred embodiments, the system is modular and multifunctional, and the user may select an appropriate probe assembly 300 which is connected at its proximal end 301 to the distal end 299 of the handle assembly 200, select a probe sheath 400 which is adapted for use with the probe assembly 300 and which is placed over the probe assembly 300 by inserting the distal end 399 of the probe assembly 300 into the proximal end 401 of the probe sheath 400 and sliding the probe sheath 400 until its proximal end 401 rests against a stopping or engagement means on the probe assembly 300 (not shown) or at its proximal end 301, and which holds it in place. The distal end 299 of the handle assembly 200 may also serve as the stopping or engagement means, and engagement means may also be located at the distal ends 399, 499 of one or both of the probe assembly 300 and the probe sheath 400. A probe tip 500 may be integrally formed with the probe sheath 400 such that the user chooses a probe sheath 400 with probe tip 500 appropriate for the particular application within the body, or the probe tip 500 may be separately chosen to be used for the particular application within the body. If the probe tip 500 is a separate element, it may be separately disposable, and may be interchanged to be used with a variety of probe sheaths 400. Such a separate probe tip 500 may be connected to the probe sheath 400 by joining the distal end 499 of the probe sheath 400 to the proximal end 501 of the probe tip. The probe tip 500 may be secured to the probe sheath 400 by any standard means known in the art including, without limitation, an interference or friction fit in which interior/exterior dimensions of the two elements are similarly sized such that they are pushed together and held in place by friction, a screw-on design in which mating threads present on each element are rotationally engaged, or a snap-on design in which flanges and bosses on each element are engaged by one or more longitudinal or rotational motions.

The control and display assembly 100 is preferably small and contains the system power supply, a computer processing unit, and user interface. The control and display assembly 100 may be powered by batteries or via a wall outlet. The unit may be placed on a tray or table near the patient. The physical size of the unit is preferably on the order of six inches wide, two inches high and four inches deep. It may be larger and have storage space for the accessory probe assemblies, probe sheaths and probe tips, or may be small enough to fit in a carrying case along with the accessories.

The handle assembly 200 preferably contains the radiation source, and the optical and electrical interconnectors to probe assemblies 300. However, the radiation source may be contained in the control and display assembly 100, and the radiation may be transmitted to the handle assembly 200 through the cable 150. Alternatively, the radiation source may be located in the probe assembly 300 or even the probe tip 500. The handle assembly 200 may also have a means to detect which type of probe assembly and/or probe sheath and/or probe tip is connected to the system and, therefore, which anatomical site, tissue type, and condition is being interrogated. An optional switch on the handle assembly 200 may activate the tissue spectroscopy or optical biopsy function, or the switch may be located on the control and display assembly 100.

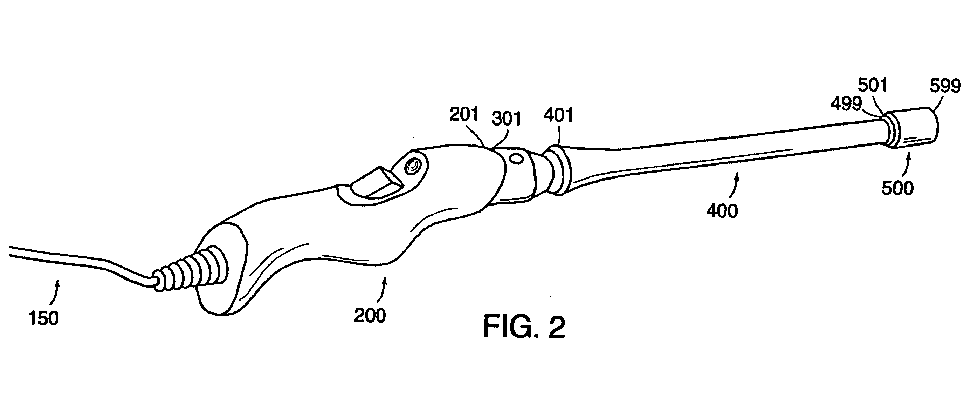

Figure 2 shows the assembled interventional device, including the cable 150 connecting the device to the control and display assembly (not shown), the handle assembly (shown with an optional control switch and indicator light) attached by its distal end 201 to the proximal end 301 of the probe assembly 300. In this particular embodiment, the proximal portion of the probe assembly 300 is visible as the proximal end 401 of the probe sheath 400 does not cover the entire probe assemble 300. The probe tip 50 is shown attached by its proximal end 501 to the distal end 499 of the probe sheath 400.

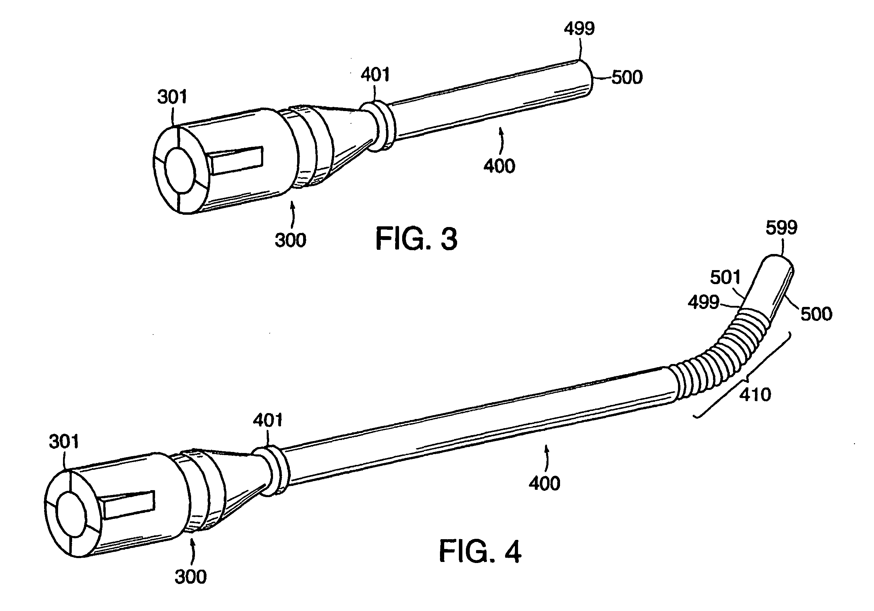

Figure 3 shows a probe assembly 300 partly ensheathed in a probe sheath 400. In this embodiment, the probe assembly 300 is relative straight and rigid. The length and diameter may, however, be varied greatly and the drawing is not to scale. In this embodiment, the probe tip 500 is shown as being substantially flat and integral with the probe sheath 400, such that the distal and proximal portions of the probe tip 500 are essentially superimposed and lie at the distal end 499 of the probe sheath 400. As before, the proximal end 401 of the probe sheath 400 is shown as being distal to the proximal end 301 of the probe assembly 300. This need not be the case, and these elements may be superimposed. The distal end 301 of the probe assembly 300 is shown disconnected, and not attached to the handle assembly 200 (not shown).

The control and display assembly 100 preferably contains at least the control, signal processing, and indication functions. The reusable probe assemblies 300 are configured as contact or proximity probes for specific applications and are directly or indirectly connectable to the control and display assembly. The reusable probes assemblies 300 are configured to access specific anatomy by conventional means, and contain at least one detector designed and configured to detect the condition of a specific tissue type for a specific anatomical site. The disposable component is configured as a probe sheath 400 and is designed for a specific probe assembly 300. The disposable probe sheath 400 provides sterility for the probe and may provide at least one functional optical characteristic. The disposable probe sheath 400 may also have interchangeable probe tips 500 that provide for differing mechanical or optical characteristics.

The system detects tissue condition by known spectrometry means, therefore, the system contains a means to illuminate (or otherwise irradiate) the tissue, a means to detect the optical response of the tissue to illumination, a means to determine the condition of the tissue by analysis of the optical response of the tissue to illumination, and a means to indicate the condition of the tissue to the user.

The means to detect the optical response of the tissue to illumination is a spectrometer module, preferably mounted near the distal tip 399 of the probe assembly 300. The spectrometer module may be configured to detect a broad continuous optical response spectrum or may be configured to detect at least one narrow optical response spectrum. The spectrometer module may contain a single photodetector or may contain an array of photodetectors. The spectrometer module may be configured to detect one or more specific spectral bands based on tissue type and condition of interest. Optical components may configure the spectrometer module for a specific application and may be in part integral with the disposable probe sheath 400. Optical components that configure the detection means to a specific application may include optical filters, spatial filters, lenses, shutters, mirrors, diffusers, or gratings.

The means to determine the condition of the tissue for a specific application is based on empirically determined probabilistic algorithms. Signals from the spectrometer module near the tip of the probe assembly 300 are processed using application-specific algorithms by a computer resident in the control and display assembly 100. Signals are sent from the spectrometer module in the probe assembly 300 to the control and display assembly 100 via electrical wires. A detection means may detect which type of probe assembly 300, and/or which type of disposable probe sheath 400 is connected to the probe thereby providing a means for automatic selection of the appropriate algorithms to be used for the specific application. The means to indicate the condition of the tissue of interest may include a simple visual binary indication, an audio indication, a graphical indication or an alpha-numeric indication. The system may include data recording, archiving and transfer means.

### Probe Assembly and Probe Sheath Designs

Probes of the general design of Figure 3 may be useful for applications including serving as a skin probe to detect the condition of the skin (e.g., to detect and distinguish between normal skin, malignant skin, and skin conditions that are precursors to malignancy) or as an ex vivo probe to detect the condition of tissue immediately upon excision and removal from the body (e.g., to detect and distinguish between various tissue conditions according to tissue type).

Figure 4 shows a probe assembly 300 partly ensheathed in a probe sheath 400. In this embodiment, the probe assembly 300 comprises a relatively straight and rigid proximal portion followed by a flexible distal portion which may be deflected within the body. The length and diameter may, however, be varied greatly and the drawing is not to scale. The probe sheath 400 is adapted to the probe assembly 300 such that it also includes a flexible portion 410 overlying the distal flexible portion of the probe assembly 300. In other embodiments, there may be multiple flexible portions of the probe assembly 300 and probe sheath 400 interspersed with relatively rigid portions. In the embodiment shown in Figure 4, the probe tip 500 is shown as having a substantially flat distal end 599, but this is spaced from the proximal end 501 which is connected to the distal end 499 of the probe sheath 400. As before, the proximal end 401 of the probe sheath 400 is shown as being distal to the proximal end 301 of the probe assembly 300. This need not be the case, and these elements may be superimposed. The distal end 301 of the probe assembly 300 is shown disconnected, and not attached to the handle assembly 200 (not shown).

Probes of the general design of Figure 4 may be useful for applications including serving as a cervical probe to detect the condition of the cervical dome and/or the endocervical canal by direct contact using palpation for guidance (e.g., to detect and distinguish between normal cervical tissue, cervical cancer, and precursor, conditions of cervical cancer).

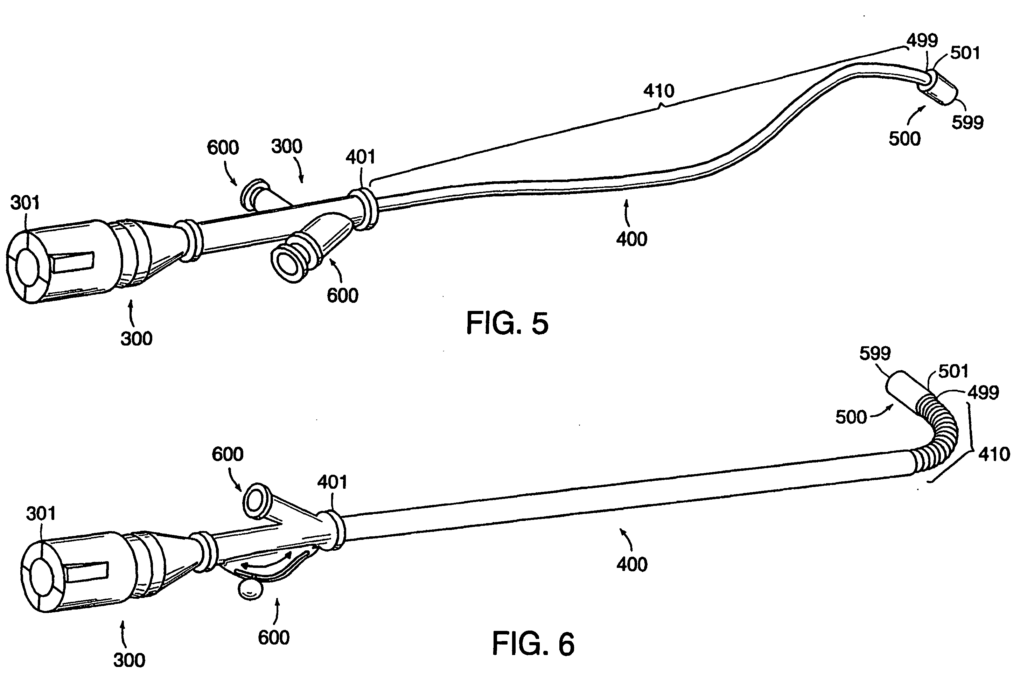

Figure 5 shows a probe assembly 300 partly ensheathed in a probe sheath 400. In this embodiment, the probe assembly 300 comprises a relatively shorter, straight and rigid proximal portion followed by a longer and flexible distal portion which may be deflected within the body. The length and diameter may, however, be varied greatly and the drawing is not to scale. The probe sheath 400 is adapted to the probe assembly 300 such that it also includes a flexible portion 410 overlying the distal flexible portion of the probe assembly 300. In other embodiments, there may be multiple flexible portions of the probe assembly 300 and probe sheath 400 interspersed with relatively rigid portions. In the embodiment shown in Figure 5, the probe tip 500 is shown as having a substantially flat distal end 599, but this is spaced from the proximal end 501 which is connected to the distal end 499 of the probe sheath 400. As before, the proximal end 401 of the probe sheath 400 is shown as being distal to the proximal end 301 of the probe assembly 300. This need not be the case, and these elements may be superimposed. The probe assembly 300 is shown as including ports 600 through which additional interventional devices may be introduced. The distal end 301 of the probe assembly 300 is shown disconnected, and not attached to the handle assembly 200 (not shown). Such a probe may also have a means to deflect the distal tip by an actuator at the proximal end.

Probes of the general design of Figure 5 may be useful for applications including serving as a hysteroscopy probe to detect the condition of the interior of the uterus (e.g., to detect and distinguish between normal uterine tissue, benign fibrous uterine cysts, uterine cancer and precursor conditions of uterine cancer, possibly guided through the working channel of a hysteroscope or by direct vision); as a bladder probe to detect the condition of the inner wall of the urinary bladder (e.g., to detect and distinguish between normal bladder wall, superficial bladder cancer, carcinoma in situ, and bladder wall conditions that are precursors to malignancy, possibly guided through the working channel of a rigid or flexible cystoscope or by direct vision); as a ureter probe to detect the condition of the ureter (e.g., to detect and distinguish between normal ureter, ureteral malignancy and ureteral conditions that are precursors to malignancy, possibly placed in the ureter by cystoscopic guidance or by percutaneous access of the renal pelvis); as a colonoscopy or sigmoidoscopy probe to detect the condition of the inner lumen of the colon (e.g., to detect and distinguish between normal colonic conditions, malignant colonic conditions, benign colonic and colonic conditions that are precursors to malignancy; possibly guided through the working channel of a colonoscope or sigmoidoscope or by direct vision); or as an esophageal probe to detect the condition of the inner lumen of the esophagus (e.g., to detect and distinguish between normal esophagus, Barrett's esophagus, esophageal malignancy and esophageal conditions that are precursors to malignancy, possibly guided through the working channel of a gastroscope or by direct vision.

Figure 6 shows a probe assembly 300 partly ensheathed in a probe sheath 400. In this embodiment, the probe assembly 300 comprises a relatively straight and rigid proximal portion followed by a flexible distal portion which may be deflected within the body. The length and diameter may, however, be varied greatly and the drawing is not to scale. The probe sheath 400 is adapted to the probe assembly 300 such that it also includes a flexible portion 410 overlying the distal flexible portion of the probe assembly 300. In other embodiments, there may be multiple flexible portions of the probe assembly 300 and probe sheath 400 interspersed with relatively rigid portions. In the embodiment shown in Figure 6, the probe tip 500 is shown as having a substantially flat distal end 599, but this is spaced from the proximal end 501 which is connected to the distal end 499 of the probe sheath 400. As before, the proximal end 401 of the probe sheath 400 is shown as being distal to the proximal end 301 of the probe assembly 300. This need not be the case, and these elements may be superimposed. The probe assembly 300 is shown as including ports 600 through which additional interventional devices may be introduced. The distal end 301 of the probe assembly 300 is shown disconnected, and not attached to the handle assembly 200 (not shown).

Probes of the general design of Figure 6 may be useful for many of the same applications as those of Figure 5, including serving as a hysteroscopy probe, as a bladder probe, as a ureter probe, as a colonoscopy or sigmoidoscopy probe, or as an esophageal probe.

Probe sheaths 400 will be configured to work with specific probe assemblies 300 and will be necessary for the proper function of the system. The probe sheath 400 and probe tip 500 will be the only components of the system that come in patient contact. The probe sheath 400 will be supplied to the user sterile, and will typically be labeled as a single-use disposable device. In a preferred embodiment the probe sheath 400 will be packaged as a component in a procedure kit, along with other single-use disposable devices normally used to access the particular anatomy of interest. In addition, the procedure kit may contain various interchangeable probe tips 500, as described below, as well as a means to test and calibrate the system for the specific application. The probe sheath 400 will be configured to have probe tips 500 that are either fixed and integral or that are interchangeable. The probe tips 500 will have optical and mechanical characteristics according to the specific application. Optical characteristics include filtering, diffusing, and provision for directional or omni-directional illumination and response detection. Mechanical characteristics include shape, firmness, lubriciousness and others required by the specific application. The probe sheath 400 may contain other mechanical features such as an ability to deflect the tip of the probe by user actuated controls.

### Probe Tip Designs

The probe tips 500 will provide optical characteristics (e.g., band pass filtering, optical diffusion, reflection, and refraction) and mechanical function as required by the specific application. Characteristics common with most applications would be optical filtering of spectral bands that are not of interest for the specific application, and optical diffusion of the illumination and detection spectrum. The probe tips 500 may be configured to remove tissue from the body or to distribute tissue about the surface of the tip for optimal system performance. The probe tips 500 may be supplied with a test and calibration cap (se below) which is removed by the user prior to tissue interrogation.

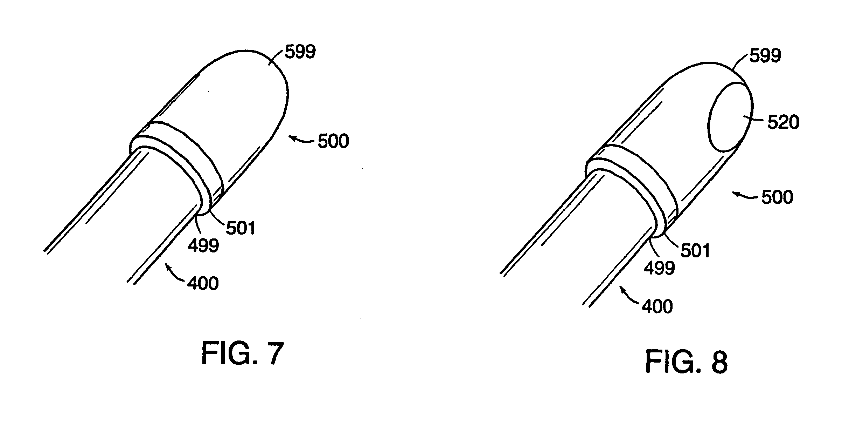

Figure 7 shows a probe tip 500 attached by its proximal end 501 to the distal end 499 of a probe sheath 400. In other embodiments, the probe sheath 400 and the probe tip 500 can be formed integrally, and both may be disposable after use. In this embodiment, the distal end of the probe tip 599 is substantially convex and designed to scatter radiation emanating from the device towards surrounding tissue, as well as to admit radiation emanating from surrounding tissue into the device. The scatter tip is a preferably designed to have optical features constructed of a solid, highly transparent material, which contains a diffuse particulate substance that provides effective Raman scattering with low optical attenuation. The scatter tip may contain materials or surfaces which provide band pass filtering, or reflection properties for light collection or direction.

Figure 8 shows a probe tip 500 attached by its proximal end 501 to the distal end 499 of a probe sheath 400. In other embodiments, the probe sheath 400 and the probe tip 500 can be formed integrally, and both may be disposable after use. In this embodiment, the distal end of the probe tip 599 is substantially opaque or impervious to the relevant radiation, but a window 520 is disposed on one side of the probe tip to allow passage of radiation emanating from the device towards adjacent tissue, as well as to admit radiation emanating from adjacent tissue into the device. The directional tip allows for tissue interrogation from a single radial segment of the probe tip. Directionality provides for concentrated and more efficient optical excitation and response-signal detection over omni-directional designs. Directionality may be accomplished using reflective or refractive means.

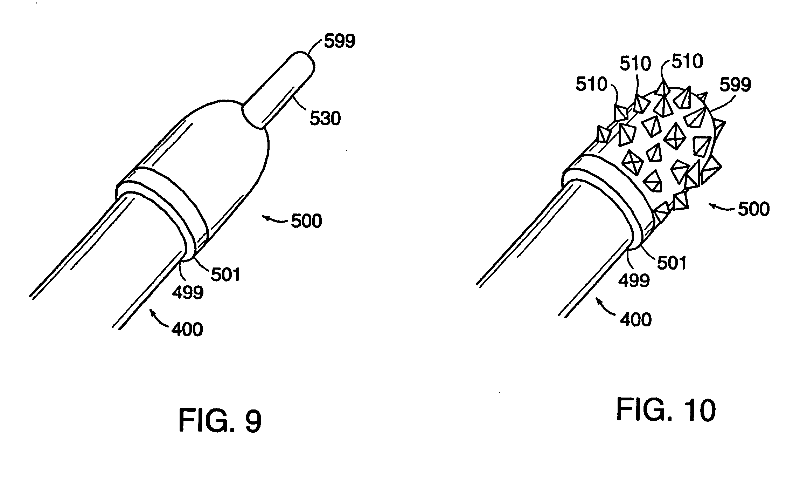

Figure 9 shows a probe tip 500 attached by its proximal end 501 to the distal end 499 of a probe sheath 400. In other embodiments, the probe sheath 400 and the probe tip 500 can be formed integrally, and both may be disposable after use. In this embodiment, the distal end of the probe tip 599 includes an extension of substantially smaller diameter than the main portion of the probe and sheath, designed for accessing smaller body cavities, orifices, perforations, incisions, or other apertures. For example, the extension 530 of such a probe tip 500 may be dimensioned specifically for use as an endocervical tip to provide atraumatic entry and tissue interrogation of the cervical canal. Optical excitation and response signals can be concentrated at the extension 530 of the tip by means of reflective coatings.

Figure 10 shows one embodiment of a probe tip 500 of the invention attached by its proximal end 501 to the distal end 499 of a probe sheath 400. In other embodiments, the probe sheath 400 and the probe tip 500 can be formed integrally, and both may be disposable after use. In this embodiment, the probe tip 500 includes a multiplicity of projections 510 which have a multiplicity of planar faces meeting at substantial angles (e.g., < 150°, preferably < 120°, more preferably < 90°). These projections 510 may operate as in a tissue "smear" to dislodge or abrade tissue samples at a site within the body. Thus, the smear tip provides removal of cellular material from the tissue interrogation site, which is then retained on the surface of the probe tip 500. The purpose may be to distribute representative target tissue cells about the surface of the probe tip to enhance the detection of a tissue condition in viva, or the smear tip may be used to sample target tissue cells for further and supplemental in vitro examination.

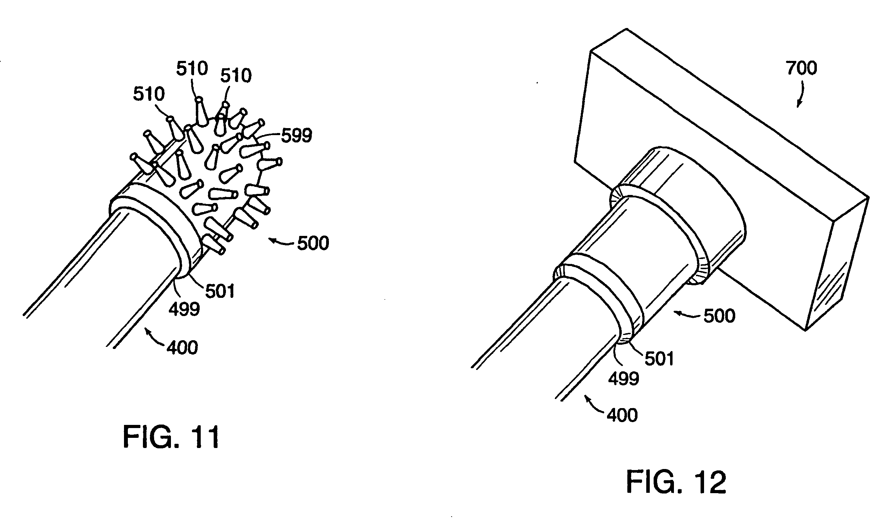

Figure 11 shows one embodiment of a probe tip 500 of the invention attached by its proximal end 501 to the distal end 499 of a probe sheath 400. In other embodiments, the probe sheath 400 and the probe tip 500 can be formed integrally, and both may be disposable after use. In this embodiment, the probe tip 500 includes a multiplicity of projections 510 which are shaped substantially as cylinders, columns or truncated conic sections which give the probe tip 500 the appearance of a "brush." The brush tip provides for sampling of target tissue cells in a manner similar to the smear tip. However, a key feature of the brush tip is that the cylinders, columns or conic sections of the brush are constructed to function as wave guides in order to concentrate the optical (or other radiation) excitation and response signals at the ends of the bristles where the removed cellular tissue resides.

In other embodiments, a "dye tip" provides for introduction of a tissue-staining dye to the site of tissue interrogation. The dye may loaded into a hydrogel coating on the probe tip 500 surface, or may be transported to the tissue interrogation sight by an irrigation channel in the probe. The probe tip 500 may have an opening in fluid communication with such a channel, or may be porous at a position overlaying the irrigation channel. The system may also include means for calibrated metering of dye onto the tissue surface. The dye may be any standard chromophore, or may be a histology reagent such as acetic acid. A dye tip may be created using essentially any design of probe tip 500, including those shown in Figures 7-11.

Figure 12 shows a probe tip 500 attached by its proximal end 501 to the distal end 499 of a probe sheath 400. In other embodiments, the probe sheath 400 and the probe tip 500 can be formed integrally, and both may be disposable after use. In this figure, the probe tip 500 is shown engaged in a calibration cap 700 which provides for system test and calibration prior to use. Such a calibration cap may be supplied with each probe tip. The surface of the calibration cap 700 in communication with the probe tip 500 is characterized by predetermined optical characteristics that provide for a system check prior to use. The calibration cap is removed prior to use.

### Optical Components

The tissue spectroscopy or optical biopsy probes preferably contain a single optical fiber which transports the laser light from a source in the control and display assembly 100, handle assembly 200, or the probe assembly itself 300, to the distal end 599 of the probe tip 500. All probes contain a spectrometer module mounted at the distal end of the probe assembly. The construction and design of the spectrometer module is application specific, however, for most applications the construction of the spectrometer module will consist of an array of silicon based photodiodes tuned to two specific tissue fluorescence bands. The individual photodiodes are tuned to specific wavelength by optical band pass filtering. The spectrometer module will have resident signal amplification and conditioning circuitry. Wires will run from the spectrometer module to the electrical interconnect at the proximal end of the probe assembly.

The illumination means may have at least one source having a broad spectral emission band such as a filament or a gas discharge lamp, or the illumination source may have at least one source with a narrow spectral emission band such as a laser or light emitting diode. The illumination spectrum applied to the tissue may have a narrower spectrum than the illumination source. The illumination light source may reside in the control and display assembly 100, in the handle assembly 200 between the control and display assembly 100 and the probe assembly 300, or in the probe assembly itself 300. The illumination light source may operate in a continuous fashion or may operate in an intermittent fashion. The operation of the illumination source may be timed to an algorithmic sequence to provide optimal or necessary system function. The illumination may be directed to the tissue of interest from a remote location by a light guide or by fiber optics.

In certain preferred embodiments, the illumination light source is a diode pumped third harmonic Nd:YAG laser. The laser operates in a pulsed mode at 10Khz, and is modulated at 30 Hz and a duty cycle of 25% to provide discrimination from ambient light and to provide continuous signal averaging as described in detail below. Average power for the laser is 1 mW at 355nm. Optics couple the laser to a single optical fiber in the probe assembly 300 which transports the light to the target tissue.

### Additional Considerations

Working Channels: The probe may be constructed with a working channel that runs from the proximal end to the distal end. The working channel allows introduction of accessory tools that may be used to treat the detected tissue conditions, or may be used to remove tissue for further diagnostic determination. Such tools may be introduced into the working channel through a port 600, and the port may pass through the probe assembly (as shown in Figures 5 and 6) or through the probe sheath as well (not shown). The distal end of the working channel may exit through the probe sheath 400 or the probe tip 500.

Irrigation Channels: The probe may contain a channel that provides for introduction of a fluid to the interrogation site. The fluid may be a simple irrigation medium such as saline, a tissue staining dye, a therapeutic agent, or a combination of fluids. Thus, the interventional devices of the invention may be used in a method of applying a tissue-staining dye to tissue in vivo, followed by interrogation of said tissue in vivo by spectroscopic means.

Cautery: The probe may provide means to cauterize tissue following the determination of the condition of said tissue.

Ambient Light Subtraction Analysis: For most or all clinical applications of this tissue spectroscopy or optical biopsy system, there will be ambient light that will, if not accounted for, interfere with the optical detection of tissue condition. A preferred means of eliminating ambient light interference is by subtraction analysis. Subtraction analysis detects the difference between two modes of operation of the system and uses the difference between the two modes as the determinant of condition. The two modes used for this system will be excitation "on" and excitation "off." During the excitation "on" phase, the target tissue will have an optical response to the excitation. During the excitation "off" phase the target tissue will not have a response to the excitation. During both modes, ambient light will be present and will be detected by the spectrometry module. By switching between the two modes at a high rate (30Hz), the system will determine tissue condition by analyzing the difference in the signal from the spectrometer module for the two modes. This will eliminate interference from ambient light conditions.

## Claims

1. A system for use with an interventional device for tissue spectroscopy or optical biopsy employing a source of radiation, comprising:
a probe sheath (400) including an open proximal end (401) and a distal end (499), and being shaped to receive the interventional device; and
a probe tip (500) disposed at the distal end (401), at least a portion of the probe tip (500) being substantially transmissive to radiation, wherein the probe tip (500) is formed integrally with the probe sheath (400) at the distal end, or, wherein the probe tip (500) is separable from and reconnectable to the distal end (499) of the probe sheath (400),
**characterised in that** said probe tip (500) comprises a multiplicity of projections (510) projecting outward from said probe tip (500).

2. The system of claim 1, wherein said multiplicity of projections (510) include a multiplicity of planar surfaces, or wherein said multiplicity of projections (510) are substantially shaped as cylinders, columns, or truncated conical sections.

3. The system of any preceding claim, wherein the portion of the probe tip (500) scatters the radiation.

4. The system of any preceding claim, wherein the probe tip (500) comprises a channel connected to a source of fluid.

5. The system of any preceding claim, wherein the probe sheath (400) is adapted to receive an interventional device selected from the group consisting of a cervical, hysterscopic, bladder, ureteral, colonoscopic, sigmoidoscopic, esophageal, and an endoscopic probe.

6. The system of any preceding claim, wherein the probe sheath (400) is disposable and/or wherein the probe tip (500) is disposable.

## Patentansprüche

1. System zur Benutzung in Verbindung mit einer Interventionalvorrichtung zur Gewebespektroskopie oder optischen Biopsie unter Benutzung einer Strahlungsquelle mit den folgenden Teilen:
eine Sondenhülle (400) mit einem offenen proximalen Ende (401) und einem distalen Ende (499), die so gestaltet ist, dass sie die Interventionalvorrichtung aufnehmen kann; und
eine Sondenspitze (500), die am distalen Ende (499) angeordnet ist, wobei wenigstens ein Teil der Sondenspitze (500) im Wesentlichen für die Strahlung durchlässig ist;
wobei die Sondenspitze (500) integral mit der Sondenhülle (400) am distalen Ende hergestellt ist;
oder wobei die Sondenspitze (500) vom distalen Ende (499) der Sondenhülle (400) abtrennbar und wieder aufsetzbar ist,
**dadurch gekennzeichnet, dass** die Sondenspitze (500) eine Vielzahl von Vorsprüngen (510) besitzt, die von der Sondenspitze (500) nach außen vorstehen.

2. System nach Anspruch 1, bei welchem die Vielzahl der Vorsprünge (510) eine Vielzahl ebener Oberflächen aufweist oder bei dem die Vielzahl der Vorsprünge (510) im Wesentlichen als Zylinder, als Säulen oder als abgestumpfte konische Abschnitte ausgebildet ist.

3. System nach einem der vorhergehenden Ansprüche, bei welchem der Abschnitt der Sondenspitze (500) die Strahlung streut.

4. System nach einem der vorhergehenden Ansprüche, bei welchem die Sondenspitze (500) einen Kanal aufweist, der mit einer Fluidquelle verbunden ist.

5. System nach einem der vorhergehenden Ansprüche, bei welchem die Sondenhülle (400) so ausgebildet ist, dass sie eine Interventionalvorrichtung aufnehmen kann, die aus der Gruppe ausgewählt ist, welche aus einer Gebärmuttersonde, einer hysterskopischen Sonde, einer Blasensonde, einer Harnröhrensonde, einer Darmsonde, einer Sigmoidoskopsonde, einer Esophagealsonde und einer endoskopischen Sonde besteht.

6. System nach einem der vorhergehenden Ansprüche, bei welchem die Sondenhülle (400) eine Wegwerfhülle ist und/oder bei welchem die Sondenspitze (500) eine Wegwerfspitze ist.

## Revendications

1. Système à utiliser avec un dispositif interventionnel pour la spectroscopie tissulaire ou la biopsie optique en utilisant une source de rayonnement, comprenant :
une gaine de sonde (400) englobant une extrémité proximale ouverte (401) et une extrémité distale (499), et conçue pour recevoir le dispositif interventionnel ; et
une pointe de sonde (500) disposée à l'extrémité distale (401), au moins une portion de la pointe de sonde (500) étant essentiellement susceptible de diffuser un rayonnement par transmission, dans lequel la pointe de sonde (500) fait partie intégrante de la gaine de sonde (400) à l'extrémité distale, ou bien dans lequel la pointe de sonde (500) pouvant être séparée et reconnectée à l'extrémité distale (499) de la gaine de sonde (400),
**caractérisé en ce que** ladite pointe de sonde (500) comprend une multitude de protubérances (510) faisant saillie à l'extérieur de ladite pointe de sonde (500).

2. Système selon la revendication 1, dans lequel ladite multitude de protubérances (510) englobe une multitude de surfaces planes ou bien dans lequel ladite multitude de protubérances (510) sont essentiellement façonnées sous la forme de cylindres, de colonnes ou de sections tronconiques.

3. Système selon l'une quelconque des revendications précédentes, dans lequel la portion de la pointe de sonde (500) diffuse le rayonnement.

4. Système selon l'une quelconque des revendications précédentes, dans lequel la pointe de sonde (500) comprend un canal connecté à une source de fluide.

5. Système selon l'une quelconque des revendications précédentes, dans lequel la gaine de sonde (400) est conçue pour recevoir un dispositif interventionnel choisi parmi le groupe constitué par une sonde cervicale, une sonde hystéroscopique, une sonde vésicale, une sonde urétérale, une sonde colonoscopique, une sonde sigmoïdoscopique, une sonde oesophagienne et une sonde endoscopique.

6. Système selon l'une quelconque des revendications précédentes, dans lequel la gaine de sonde (400) est jetable et/ou dans lequel la pointe de sonde (500) est jetable.
